# EUROPEAN PATENT SPECIFICATION

(11) **EP 2 934 521 B1**
(45) Date of publication and mention of the grant of the patent: **30.11.2016**
(21) Application number: 12812221.5
(22) Date of filing: 19.12.2012
(51) Int. Cl.: A61K 31/427, A61K 45/06, A61K 38/05, A61P 17/06

(54) **USE OF PIDOTIMOD TO TREAT PSORIASIS**
VERWENDUNG VON PIDOTIMOD ZUR BEHANDLUNG VON PSORIASIS
UTILISATION DU PIDOTIMOD POUR TRAITER LE PSORIASIS

(43) Date of publication of application: 28.10.2015
(73) Proprietor: Polichem SA, 1526 Luxembourg (LU)
(72) Inventor: CASERINI, Maurizio, I-22100 Como (IT); MAILLAND, Federico, CH-6900 Lugano (CH)
(74) Representative: Pistolesi, Roberto
(86) International application number: PCT/EP2012/076088
(87) International publication number: WO 2014/094840

(56) References cited:
- IT-B- 1 231 723
- GOURGIOTIS DIMITRIOS ET AL: "Immune modulator pidotimod decreases the in vitro expression of CD30 in peripheral blood mononuclear cells of atopic asthmatic and normal children", JOURNAL OF ASTHMA, ASTHMA PUBLICATIONS SOCIETY, OSSINING, NY, US, vol. 41, no. 3, 1 January 2004 (2004-01-01), pages 285-287, XP008164025, ISSN: 0277-0903, DOI: 10.1081/JAS-120026085
- POZZI E ET AL: "Pidotimod in the treatment of patients affected by bacterial exacerbations of chronic bronchitis", 19940101, vol. 44, no. 12A, 1 January 1994 (1994-01-01), pages 1495-1498, XP008097244,

## Description

The present invention is directed to the use of pidotimod, or a physiologically acceptable salt thereof, to treat psoriasis.

### BACKGROUND OF THE INVENTION

Psoriasis is a common, chronic, multi-system disease with predominantly skin and joint manifestations affecting approximately 2% of the population. The major manifestation of psoriasis is chronic lesion of the skin. It is characterized by disfiguring, scaling, and erythematous plaques that may be painful or often severely pruritic and may cause significant quality of life issues. Psoriasis is a chronic disease that waxes and wanes during a patient's lifetime, is often modified by treatment initiation and cessation and has few spontaneous remissions. More severe localizations of psoriasis include psoriatic arthritis, nail psoriasis and enthesitis, i.e. an autoimmune lesion linking nail and joint involvement in psoriatic disease.

Psoriasis is a complex genetic disease of dysregulated immune function, although the mechanism of inheritance has not been completely defined. A number of environmental factors play an important role in the pathogenesis of psoriasis, including drugs, skin trauma (Koebner's phenomenon), infection, and stress.

Evidence suggesting that psoriasis involves immunologic mechanisms includes the efficacy of immunosuppressive drugs such as methotrexate, cyclosporine (CyA), immune-targeting biologic agents, and immunotoxins (denileukin diftitox).

Thus, psoriasis is an hyperimmune-mediated organ-specific (skin, nails and/or joints) disease in which local lesion primes basal stem keratinocytes to hyperproliferate and perpetuate the disease process.

In the art, pharmacologic treatment of psoriasis is done by systemic or by topical treatment. Systemic treatments are needed to treat severe skin lesions and/or extra-skin localizations of the disease, such as psoriatic arthritis and nail psoriasis. Systemic traditional treatments are performed by use of methotrexate, cyclosporine, corticosteroids, retinoids, or fumaric acid esters. Methotrexate, cyclosporine and corticosteroids act mostly by depressing the immune response, while retinoids and fumaric acid esters act primarily by inhibiting the hyperproliferation of the keratinocytes which is at the basis of the psoriatic lesions. None of those agents is devoid of toxic effects and the efficacy is limited. More recently, biologics have been introduced, including alefacept, etanercept, and a number of monoclonal antibodies, namely adalimumab, infliximab, ustekinumab. The efficacy of such new agents is improved compared to the traditional systemic agents, but they need to be taken for a long time, the cost of therapy is very high and severe adverse events may be associated to the therapy.

Topical treatment is reserved to cases where the disease is localized to skin only. In the art, topical treatment of psoriasis is made made by anthralin, Vitamin D3 and analogues, including calcipotriol and calcitriol, tazarotene, which is a topical retinoid, and topical glucocorticoids. Topic products are effective and safe, though their use is limited in case of large body surface affected. Patients candidate to topical therapies should present an established clinical diagnosis of mild to moderate psoriasis with a body surface involvement ≤ 10% or an index of severity, measured by means of PASI calculation, ≤ 10.

Psoriasis Area and Severity Index (PASI) is the most widely used tool for the measurement of severity of psoriasis. PASI combines the assessment of the severity of lesions and the area affected into a single score in the range 0 (no disease) to 72 (maximal disease) (Langley RG, Ellis CN. J Am Acad Dermatol 2004; 51: 563-9). Whichever the treatment, the effect is only on clinical symptoms and does not last very long. Moreover, long lasting treatments are limited by the strong toxicity. Thus, there is still an unsatisfied medical need in terms of efficacy and prevention of relapses. Pidotimod, whose chemical name is (4*R*)-3-(5-oxo-L-prolyl)-1,3-thiazolidine-4-carboxylic acid, is a synthetic drug known for its capability to increase the immune response in animal models and in human beings; it was disclosed for the first time in IT1231723. In vitro studies both from animal and human specimens have documented a good activity on innate and adaptive immune responses and have been confirmed by in vivo clinical studies, demonstrating the efficacy of pidotimod in reducing the rate of recurrent infections of the upper respiratory and urinary tract in children. Same results were obtained in recurrent respiratory tract infections in adults. More importantly, these effects are more evident in setting of immune defects such as senescence, Down syndrome, surgery, and cancer (Riboldi et al. Int J Immunopathol Pharmacol. 2009; 22(2): 255-62). Due to its capability to stimulate the immune system, pidotimod is believed to worsen those conditions characterized by an increased immune activity and its use is not recommended in such diseases.

Contrary to any expectation, it has now been surprisingly found that pidotimod, besides being active on illnesses characterized by immune defects, may be of benefit in patients with psoriasis, by attenuating the skin and enthesis lesions typical of such a disease.

### DESCRIPTION OF THE INVENTION

The object of the present invention is represented by the use of pidotimod, or a physiologically acceptable salt thereof, for use in the treatment of psoriasis.

For the treatment of the present invention, pidotimod, or a physiologically acceptable salt thereof, may be administered either systemically or topically.

When administered systemically, it may be in the form of solid or liquid formulations containing pidotimod or a physiologically acceptable salt thereof together with at least a pbarmaceutically acceptable excipient and/or adjuvant; such formulations may be in the form of tablets, film-coated tablets, capsules, dragees, sachets, solutions or suspensions. Such liquid formulations to be systemically administered may have a w/w concentration in pidotimod from 0.5% to 20%, more preferably from 1% to 10%, most preferably from 2% to 8%.

Such solid formulations to be systemically administered may have a w/w concentration in pidotimod from 50% to 90%, more preferably from 65% to 80%, most preferably from 70% to 75%.

According to an embodiment of the invention, when administered systemically, the amount of pidotimod or of a physiologically acceptable salt thereof, may vary from 10 to 1000 mg per single dose, more preferably from 50 to 800 mg per single dose.

Such solid, semi-solid or liquid formulations are particularly suitable to treat psoriasis in all its manifestations, including skin psoriasis, nail psoriasis, psoriatic arthritis. When topically administered, pidotimod, or a physiologically acceptable salt thereof, may be in the form of semi-solid or liquid formulations containing pidotimod or a physiologically acceptable salt thereof, together with at least a pharmaceutically acceptable excipient and/or adjuvant; such formulations may be in the form of solutions, emulsions or suspensions, creams, gels and ointments.

Such semi-solid or liquid formulations to be topically administered may have a w/w concentration in pidotimod from 0.1% to 20%, more preferably from 1% to 15%, most preferably from 5% to 10%. They are particularly suitable to treat skin psoriasis by direct application over the skin lesions.

Pharmaceutical compositions may be prepared according to conventional techniques, may contain pharmaceutically acceptable excipients, adjuvants and/or carriers, and may also contain, in combination, one or more active principles with complementary or, in any case, useful activity.

The active agents which may be used in combination with pidotimod of the present invention include, but are not limited to, immunosuppressive agents, Vitamin D and analogues, Vitamin A related compounds, corticosteroids, biologics; such active ingredients may be administered together with pidotimod (i.e. they may be for instance contained in the same composition as pidotimod) or they may be administered separately from or in temporal proximity with pidotimod, either by systemic (oral, intravenous, intramuscular) route or by topical route, directly on the skin or nail lesions. Examples of immunosuppressive agents include methotrexate, azathioprine, cyclosporine, fumaric acid, tacrolimus or pimecrolimus and corticosteroids; examples of Vitamin D analogues include calcitriol, calcipotriol and tacalcitol: examples of Vitamin A related compounds include retinoids, tretinoine, isotretinoine, etretinate, acitretine, tazarotene, bexarotene and adapalene; examples of biologies include alefacept, etmercept, and monoclonal antibodies adalimumab, infliximab, ustekinumab. Examples of the compositions prepared according to the present invention include: tablets, film-coated tablets, capsules, dragees or syrup suitable for oral administration, ampoules and vials for intramuscular or intravenous administration; cream, gel, ointment, solution, emulsion, suspension for topical application.

The pharmaceutical compositions and the uses of the present invention will now be more fully described by the following examples.

### EXAMPLE 1

An oil in water cream having the following w/w % composition was prepared:

| | |
|---|---|
| 1. Pidotimod | 10.00% |
| 2. Tris(hydroxymethyl)methylamine* | 5.20% |
| 3. Lactic Acid | 0.20% |
| 4. Disodium EDTA | 0.10% |
| 5. Glycerin | 5.00% |
| 6. Xanthan Gum | 0.25% |
| 7. Hydroxypropyl Chitosan | 0.50% |
| 8. Emulsifiers | 15.50% |
| 9. Medium chain Triglycerides | 3.00% |
| 10. 2-Octyldodecyl Alcohol | 2.00% |
| 11. Diethylene Glycol Monoethyl Ether | 5.00% |
| 12. DL-Alpha Tocopheryl Acetate | 0.50% |
| 13. Decamethylcyclopentasiloxane | 3.00% |
| 14. Preservatives | 1.00% |
| 15. Purified Water | q.s. to 100.00% |

| | |
|---|---|
| * tromethamine | |

### Preparation

In the main vessel, solubilize components 1, 2, 3, 4, 5 in part of water. Add Xanthan Gum and disperse thoroughly until homogeneity. Separately solubilize component 7 in part of water, then add it to the main vessel while stirring. Heat the phase at 70 - 75°C. In another vessel combine the components 8, 9, 10, 11, 12 and heat at 70 - 75°C while stirring. Combine the two phases heated at the same temperature and homogenize for about 10 minutes. Cool down to 40° and add on sequence components 13 and 14, homogenizing after each addition.
Cool down to room temperature under moderate stirring.

### EXAMPLE 2

A topical solution having the following w/w % composition was prepared:

| | |
|---|---|
| 1. Pidotimod | 10.00% |
| 2. Tris(hydroxymethyl)methylamine | 5.00% |
| 3. Disodium EDTA | 0.10% |
| 4. Propylene Glycol | 5.00% |
| 5. Lactic acid | 0.15% |
| 6. Hydroxypropyl Chitosan | 1.00% |
| 7. Purified water | q.s. to 100.00% |

### Preparation

Solubilize components 1, 2, 3, 4, 6 in water. Add component 7 and mix until clear solution is obtained.

### EXAMPLE 3

A detergent body and scalp formulation having the following w/w % composition was prepared:

| | |
|---|---|
| 1. Pidotimod | 5.00% |
| 2. Tris(hydroxymethyl)methylamine | 2.50% |
| 3. Purified water | q.s to 100.00% |
| 4. Hydroxypropyl Chitosan | 1.500% |
| 5. Surfactants | 43.00% |
| 6. Citric Acid Monohydrate | 0.30% |
| 7. Sodium Chloride | 1.00% |
| 8. Benzyl alcohol | 1.00% |
| 9. Diethyleneglycol Lauryl Ether | 2.00% |

### Preparation

In the main vessel combine the surfactant mixture 5. Add component 8 and solubilize until clear solution. Add component 9 and mix until homogeneity. Separately, in part of water, solubilize components 1, 2, 4, 6 and add it in the main vessel while stirring. Finally regulate viscosity adding component 7. Mix until clear solution.

### EXAMPLE 4

A topical gel formulation having the following w/w % composition was prepared:

| | |
|---|---|
| 1. Purified water | q.s to 100.00% |
| 2. Pidotimod | 10.00% |
| 3. Tris(hydroxymethyl)methylamine | 5.00% |
| 4. Disodium Edta | 0.10% |
| 5. Glycerin | 5.00% |
| 6. 5-Ureidohydantoin | 0.30% |
| 7. Thickeners | 0.80% |
| 8. Hydroxypropyl Chitosan | 0.50% |
| 9. Preservatives | 0.33% |

### Preparation

In the main vessel combine the components 1, 2, 3, 4, 5, 6, and 9. Mix until clear solution. Add thickeners homogenizing after each addition and until fully dispersed. Separately solubilize component 8 in part of water and add it in the main vessel while stirring. Mix until homogeneity.

### EXAMPLE 5

A granulate for oral administration having the following w/w % composition was prepared:

| | |
|---|---|
| 1. Pidotimod | 26.67% |
| 2. Mannitol | 3.33% |
| 3. Binder and wetting agent | 0.90% |
| 4. Sweetener | 0.60% |
| 5. Flavour | 16.67% |
| 6. Sodium carbonate anhydrous | 5.67% |
| 7. Silicon dioxide | 0.33% |
| 8. Colouring agents | 0.04% |
| 9. Saccharose | q.s. to 100% |

### Preparation

In a vessel dissolve the component 3 in a suitable quantity of water. Mix until clear solution. In another vessel mix the components 1 and 2. Spray the obtained solution onto mixed components until a homogeneous granulate is obtained. After drying, components from 4 to 9 are added to the obtained granulate. All components are mixed until an homogeneous mixture is obtained.

### EXAMPLE 6

An injectable solution having the following w/V composition was prepared:

| | |
|---|---|
| 1. Pidotimod | 220 mg |

| | |
|---|---|
| 2. Buffering agents | 1.65 mg |
| 3. Tromethamine | q.s. to pH 6.5 |
| 4. Water for injections | q.s. to 3.3 mL |

Preparation: the components 1 to 3 are dissolved in water for injections and mixed until a homogeneous solution is obtained with a pH of 6.5.

### EXAMPLE 7

A solution for oral administration having the following w/w % composition was prepared:

| | |
|---|---|
| 1. Pidotimod | 5.10% |
| 2. Sodium chloride | 0.07% |
| 3. Sodium saccharin | 0.06% |
| 4. Chelating agents | 0.05% |
| 5. Tromethamine | 2.50% |
| 6. Preservatives | 0.15% |
| 7. Sorbitol solution | 31.89% |
| 8. Flavouring agents | 0.30% |
| 9. Antioxydants | 0.07% |
| 10. Colouring agents | 0.01% |
| 11. Purified water | 59.80% |

Preparation: in a vessel dissolve the components 1 to 10 in a suitable quantity of purified water. Mix until a clear solution is obtained. Add the remaining quantity of water, mix until a homogeneous solution is obtained and filter.

### EXAMPLE 8

A tablet for oral administration having the following w/w % composition was prepared:

| | |
|---|---|
| 1. Pidotimod | 72.70% |
| 2. Diluents | 17.65% |
| 3. Sodium Carboxymethyl cellulose crosslinked | 4.55% |
| 4. Binders | 4.00% |
| 5. Magnesium stearate | 1.10% |

In a vessel mix the components 1 and 2. In another vessel dissolve the component 4 in a suitable quantity of water. Mix until a clear solution is obtained. Spray the obtained solution onto mixed components 1 and 2 until a homogeneous granulate is obtained. After drying, components 3 and 5 are added to the obtained granulate and mixed until a homogeneous mixture is obtained. The mixture is then compressed by means of a tableting machine.

### EXAMPLE 9

An evaluation of the activity of pidotimod was tested on psoriatic patients, in order to assess the efficacy in terms of improvement of the pathology by means of PASI evaluation. The safety of the treatment was also evaluated. The study was performed in 5 patients (3 males and 2 females, aged between 31 - 53, mean = 42) with a clinical diagnosis of mild psoriasis of at least 6 months. The patients, before and during pidotimod treatment, did not take concomitant treatments with local corticosteroids or systemic therapy. The body surface area involvement at the baseline was ≤10% with a PASI value ≤10.

The study product was taken with the composition of the Example 6 at a dosage of two oral vials daily, i.e. 800 mg daily of the active ingredient, taken far from mealtimes. During the trial, the following visits were performed:
- baseline - T0 (before the product use)
- intermediate visit - T6 (after 6 weeks of treatment)
- final visit - T12 (after 12 weeks of treatment)

No relevant event, which may have interfered to the test results, occurred during the study period.

The efficacy of the product was expressed by mean of values of PASI evaluation, assessing the PASI at the baseline and at the intermediate visit and the final one. The results were reported in the table below:

| **Age/Sex (M,F)** | **Plaques Localizati on** | **History of Psoriasis** | **Concomitant Treatments** | **PASI Evaluation** | | |
|---|---|---|---|---|---|---|
| | | | | **Baseline Mean: 3.22 SD: 0.68** | **6 weeks Mean: 2.78 SD: 0.62** | **12 weeks Mean: 2.32 SD: 0.54** |
| 36 M | Head, arms | 8 yrs | Topical calcipotriol | 3.2 | 2.9 | 2.4 |
| 53 M | Knees | 13 yrs | - | 2.3 | 2.0 | 1.8 |
| 48 F | Knees, pubes | 4 yrs | - | 3.1 | 2.6 | 2 |
| 31 F | Arms, knees | 3 yrs | Topical calcipotriol | 3.3 | 2.7 | 2.2 |
| 42 M | Head, arms, knees | 9 yrs | - | 4.2 | 3.7 | 3.2 |

The mean value of PASI at baseline was 3.22 with a standard deviation (SD) of 0.68; at the intermediate visit, the mean of the PASI values was 2.78 (SD = 0.62), while at the end of treatment the mean of PASI value was 2.32 (SD = 0.54). The obtained results showed that the study product determined a statistically significant decrease (Student t test p < 0.05) of the PASI value at T12 (end of treatment) vs. T0 (baseline).

Besides the decrease in the PASI values, an important improvement in the clinical evidences of the illness has been shown in all patients. Moreover, the treatment was very well tolerated and no side effects were reported.

In conclusions, the treatment with pidotimod (800mg/die) was able to improve the PASI values, index needed to measure the severity and extent of psoriasis with a result, at the end of the treatment, lasted 12 weeks, statistically significant (Student t test p < 0.05), compared to the baseline and it suggests the use of pidotimod in the treatment of psoriasis vulgaris, mild to moderate.

## Claims

1. Pidotimod or a physiologically acceptable salt thereof, for use in the treatment of psoriasis.

2. Pidotimod or a physiologically acceptable salt thereof for use according to claim 1, **characterized in that** said psoriasis is skin psoriasis, nail psoriasis or psoriatic arthritis.

3. Pidotimod or a physiologically acceptable salt thereof for use according to claim 1, **characterized in that** it is administered to a human.

4. Pidotimod or a physiologically acceptable salt thereof for use according to claim 1, **characterized in that** it is administered systemically.

5. Pidotimod or a physiologically acceptable salt thereof for use according to claim 4, **characterized in that** it is administered by means of a solid or liquid formulation.

6. Pidotimod or a physiologically acceptable salt thereof for use according to claim 5, **characterized in that** said solid formulation is a tablet, a film-coated tablet, a capsule, a dragée or a sachet.

7. Pidotimod or a physiologically acceptable salt thereof for use according to claim 5, **characterized in that** said liquid formulation is a solution or a suspension.

8. Pidotimod or a physiologically acceptable salt thereof for use according to claim 5, **characterized in that** said solid formulation has a w/w concentration in pidotimod from 50% to 90%, more preferably from 65% to 80%, most preferably from 70% to 75%.

9. Pidotimod or a physiologically acceptable salt thereof for use according to claim 5, **characterized in that** said liquid formulation has a w/w concentration in pidotimod from 0.5% to 20%, more preferably from 1% to 10%, most preferably from 2% to 8%.

10. Pidotimod or a physiologically acceptable salt thereof for use according to claim 5, **characterized in that** said formulation has a content in pidotimod or a salt thereof, from 10 to 1000 mg per single dose, preferably from 50 to 800 mg per single dose.

11. Pidotimod or a physiologically acceptable salt thereof for use according to claim 1, **characterized in that** it is administered topically.

12. Pidotimod or a physiologically acceptable salt thereof for use according to claim 11, **characterized in that** it is administered by means of a semi-solid or liquid formulation.

13. Pidotimod or a physiologically acceptable salt thereof for use according to claim 12, **characterized in that** semi-solid formulation is a cream, a gel, an ointment or an emulsion.

14. Pidotimod or a physiologically acceptable salt thereof for use according to claim 12, **characterized in that** said liquid formulation is a solution or a suspension.

15. Pidotimod or a physiologically acceptable salt thereof for use according to claim 12, **characterized in that** said formulation has a w/w concentration in pidotimod, or a salt thereof from 0.1% to 20%, preferably from 1% to 15%, more preferably from 5% to 10%.

16. Pidotimod or a physiologically acceptable salt thereof for use according to any of the preceding claims, **characterized in that** it is administered in combination or in temporal proximity with at least one additional active principle, wherein said at least one additional active principle is selected from immunosuppressive agents, preferably methotrexate, azathioprine, cyclosporine, fumaric acid, tacrolimus or pimecrolimus and corticosteroids, Vitamin D and analogues, preferably calcitriol, calcipotriol and tacalcitol, Vitamin A related compounds, preferably retinoids, tretinoine, isotretinoine, etretinate, acitretine, tazarotene, bexarotene and adapalene, biologics, preferably alefacept, etanercept, and monoclonal antibodies adalimumab, infliximab, ustekinumab.

## Patentansprüche

1. Pidotimod oder ein physiologisch akzeptables Salz davon, zur Verwendung in der Behandlung von Psoriasis.

2. Pidotimod oder ein physiologisch akzeptables Salz davon zur Verwendung gemäß Anspruch 1, **dadurch gekennzeichnet, dass** die Psoriasis Haut-Psoriasis, Nägel-Psoriasis oder psoriatische Arthritis ist.

3. Pidotimod oder ein physiologisch akzeptables Salz davon zur Verwendung gemäß Anspruch 1, **dadurch gekennzeichnet, dass** es einem Menschen verabreicht wird.

4. Pidotimod oder ein physiologisch akzeptables Salz davon zur Verwendung gemäß Anspruch 1, **dadurch gekennzeichnet, dass** es systemisch verabreicht wird.

5. Pidotimod oder ein physiologisch akzeptables Salz davon zur Verwendung gemäß Anspruch 4, **dadurch gekennzeichnet, dass** es mittels einer festen oder flüssigen Formulierung verabreicht wird.

6. Pidotimod oder ein physiologisch akzeptables Salz davon zur Verwendung gemäß Anspruch 5, **dadurch gekennzeichnet, dass** diese feste Formulierung eine Tablette, eine Filmbeschichtete Tablette, eine Kapsel, ein Dragee oder ein Sachet ist.

7. Pidotimod oder ein physiologisch akzeptables Salz davon zur Verwendung gemäß Anspruch 5, **dadurch gekennzeichnet, dass** die flüssige Formulierung eine Lösung oder eine Suspension ist.

8. Pidotimod oder ein physiologisch akzeptables Salz davon zur Verwendung gemäß Anspruch 5, **dadurch gekennzeichnet, dass** diese feste Formulierung eine Gew./Gew.-Konzentration an Pidotimod von 50% bis 90%, mehr bevorzugt von 65% bis 80%, am meisten bevorzugt von 70% bis 75% aufweist.

9. Pidotimod oder ein physiologisch akzeptables Salz davon zur Verwendung gemäß Anspruch 5, **dadurch gekennzeichnet, dass** diese flüssige Formulierung eine Gew./Gew.-Konzentration an Pidotimod von 0,5% bis 20%, mehr bevorzugt von 1% bis 10%, am meisten bevorzugt von 2% bis 8% aufweist.

10. Pidotimod oder ein physiologisch akzeptables Salz davon, zur Verwendung gemäß Anspruch 5, **dadurch gekennzeichnet, dass** diese Formulierung einen Gehalt an Pidotimod oder einem Salz davon von 10 bis 1000 mg pro Einzeldosis, bevorzugt von 50 bis 800 mg pro Einzeldosis, aufweist.

11. Pidotimod oder ein physiologisch akzeptables Salz davon zur Verwendung gemäß Anspruch 1, **dadurch gekennzeichnet, dass** es topisch verabreicht wird.

12. Pidotimod oder ein physiologisch akzeptables Salz davon, zur Verwendung gemäß Anspruch 11, **dadurch gekennzeichnet, dass** es mittels einer halbfesten oder flüssigen Formulierung verabreicht wird.

13. Pidotimod oder ein physiologisch akzeptables Salz davon zur Verwendung gemäß Anspruch 12, **dadurch gekennzeichnet, dass** die halbfeste Formulierung eine Creme, ein Gel, eine Salbe oder eine Emulsion ist.

14. Pidotimod oder ein physiologisch akzeptables Salz davon zur Verwendung gemäß Anspruch 12, **dadurch gekennzeichnet, dass** diese flüssige Formulierung eine Lösung oder eine Suspension ist.

15. Pidotimod oder ein physiologisch akzeptables Salz davon zur Verwendung gemäß Anspruch 12, **dadurch gekennzeichnet, dass** diese Formulierung eine Gew./Gew.-Konzentration an Pidotimod oder einem Salz davon von 0,1% bis 20%, bevorzugt von 1% bis 15%, mehr bevorzugt von 5% bis 10%, aufweist.

16. Pidotimod oder ein physiologisch akzeptables Salz davon zur Verwendung gemäß irgendeinem der vorhergehenden Ansprüche, **dadurch gekennzeichnet, dass** es in Kombination mit oder in zeitlicher Nähe mit mindestens einem zusätzlichen Wirkstoff verabreicht wird, wobei dieser mindestens eine zusätzliche Wirkstoff ausgewählt ist aus immuno-suppressiven Agentien, bevorzugt Methotrexat, Azatioprin, Cyclosporin, Fumarsäure, Tacrolimus oder Pimecrolimus und Corticosteroiden, Vitamin D und Analoga, bevorzugt Calcitriol, Calcipotriol und Tacalcitol, Vitamin A-verwandte Verbindungen, bevorzugt Retinoide, Tretinoin, Isotretinoin, Etretinat, Acitretin, Tazaroten, Bexaroten und Adapalen, biologische Präparate, bevorzugt Alefacept, Etanercept, und monoklonalen Antikörpern Adalimumab, Inflixmab, Ustekinumab.

## Revendications

1. Pidotimod ou un sel physiologiquement acceptable de celui-ci, pour utilisation dans le traitement du psoriasis.

2. Pidotimod ou un sel physiologiquement acceptable de celui-ci pour utilisation selon la revendication 1, **caractérisé en ce que** ledit psoriasis est un psoriasis de la peau, un psoriasis des ongles ou un rhumatisme psoriasique.

3. Pidotimod ou un sel physiologiquement acceptable de celui-ci pour utilisation selon la revendication 1, **caractérisé en ce qu'**il est administré à un être humain.

4. Pidotimod ou un sel physiologiquement acceptable de celui-ci pour utilisation selon la revendication 1, **caractérisé en ce qu'**il est administré de manière systémique.

5. Pidotimod ou un sel physiologiquement acceptable de celui-ci pour utilisation selon la revendication 4, **caractérisé en ce qu'**il est administré à l'aide d'une formulation solide ou liquide.

6. Pidotimod ou un sel physiologiquement acceptable de celui-ci pour utilisation selon la revendication 5, **caractérisé en ce que** ladite formulation solide est un comprimé, un comprimé pelliculé, une capsule, une dragée ou un sachet.

7. Pidotimod ou un sel physiologiquement acceptable de celui-ci pour utilisation selon la revendication 5, **caractérisé en ce que** ladite formulation liquide est une solution ou une suspension.

8. Pidotimod ou un sel physiologiquement acceptable de celui-ci pour utilisation selon la revendication 5, **caractérisé en ce que** ladite formulation solide a une concentration en p/p de pidotimod de 50 % à 90 %, plus préférablement de 65 % à 80 %, de manière préférée entre toutes de 70 % à 75 %.

9. Pidotimod ou un sel physiologiquement acceptable de celui-ci pour utilisation selon la revendication 5, **caractérisé en ce que** ladite formulation liquide a une concentration en p/p de pidotimod de 0,5 % à 20 %, plus préférablement de 1 % à 10 %, de manière préférée entre toutes de 2 % à 8 %.

10. Pidotimod ou un sel physiologiquement acceptable de celui-ci pour utilisation selon la revendication 5, **caractérisé en ce que** ladite formulation a une teneur en pidotimod ou en un sel de celui-ci, de 10 à 1 000 mg par dose individuelle, de préférence de 50 à 800 mg par dose individuelle.

11. Pidotimod ou un sel physiologiquement acceptable de celui-ci pour utilisation selon la revendication 1, **caractérisé en ce qu'**il est administré par voie topique.

12. Pidotimod ou un sel physiologiquement acceptable de celui-ci pour utilisation selon la revendication 11, **caractérisé en ce qu'**il est administré à l'aide d'une formulation semi-solide ou liquide.

13. Pidotimod ou un sel physiologiquement acceptable de celui-ci pour utilisation selon la revendication 12, **caractérisé en ce que** la formulation semi-solide est une crème, un gel, un onguent ou une émulsion.

14. Pidotimod ou un sel physiologiquement acceptable de celui-ci pour utilisation selon la revendication 12, **caractérisé en ce que** ladite formulation liquide est une solution ou une suspension.

15. Pidotimod ou un sel physiologiquement acceptable de celui-ci pour utilisation selon la revendication 12, **caractérisé en ce que** ladite formulation a une concentration en p/p de pidotimod ou d'un sel de celui-ci de 0,1 % à 20 %, de préférence de 1 % à 15 %, plus préférablement de 5 % à 10 %.

16. Pidotimod ou un sel physiologiquement acceptable de celui-ci pour utilisation selon l'une quelconque des revendications précédentes, **caractérisé en ce qu'**il est administré en association avec au moins un principe actif additionnel, ou à proximité temporelle de celui-ci, dans lequel ledit au moins un principe actif additionnel est choisi parmi les agents immunosuppresseurs, de préférence le méthotrexate, l'azathioprine, la cyclosporine, l'acide fumarique, le tacrolimus ou le pimécrolimus et les corticostéroïdes, la vitamine D et analogues, de préférence le calcitriol, le calcipotriol et le tacalcitol, les composés associés à la vitamine A, de préférence les rétinoïdes, la trétinoïne, l'isotrétinoïne, l'étrétinate, l'acitrétine, le tazarotène, le bexarotène et l'adapalène, les produits biologiques, de préférence l'aléfacept, l'étanercept, et les anticorps monoclonaux adalimumab, infliximab, ustekinumab.
